Europäisches Patentamt

European Patent Office

Office européen des brevets

(19)

(11) Publication number: **0 072 599**
**A1**

(12)
# EUROPEAN PATENT APPLICATION

(21) Application number: **82201023.7**

(22) Date of filing: **13.08.82**

(51) Int. Cl.³: **C 07 C 13/18**
**C 07 C 13/10**

(30) Priority: **19.08.81 NL 8103871**

(43) Date of publication of application:
**23.02.83 Bulletin 83/8**

(84) Designated Contracting States:
**BE CH DE FR GB IT LI NL**

(71) Applicant: **STAMICARBON B.V.**
**Postbus 10**
**NL-6160 MC Geleen(NL)**

(72) Inventor: **Plantema, Otto Gerrit**
**Braakpeel 1**
**NL-6034 RP Nederweert Eind(NL)**

(72) Inventor: **Roberts, Mathieu Johannes A.**
**Vonkstraat 19**
**NL-6436 BK Amstenrade(NL)**

(74) Representative: **Pinckaers, August René et al,**
**OCTROOIBUREAU DSM Postbus 9**
**NL-6160 MA Geleen(NL)**

(54) **Process for the preparation of cyclohexane and methylcyclopentane.**

(57) Process for preparing cyclohexane and methylcyclopentane from the distillation residue obtained in a cylohexane oxidation process or a phenol hydrogenation process by hydrogenating such a residue in the presence of a hydro-cracking catalyst.

EP 0 072 599 A1

-1-

## PROCESS FOR THE PREPARATION OF CYCLOHEXANE AND METHYLCYCLOPENTANE

The invention relates to a process for the preparation of cyclohexane and methylcyclopentane.

Cyclohexane is a raw material much used for the preparation of ε-caprolactam. To this end the cyclohexane is oxidized to form a mixture of cyclohexanone and cyclohexanol, which is separated by distillation. The cyclohexanone is then converted, via the oxime, into ε-caprolactam, while the cyclohexanol is dehydrogenated to form a mixture of cyclohexanone and cyclohexanol, which is added to the originally acquired mixture of cyclohexanone and cyclohexanol to be separated by distillation. Such a process is known, for instance, from the United States patent specification 3,946,076.

A mixture containing cyclohexanone and cyclohexanol can be obtained also by hydrogenating phenol (see, for instance, the United States patent specification 2,873,296). By separating this mixture by distillation, dehydrogenating the cyclohexanol and recirculating the mixture of cyclohexanone and cyclohexanol obtained in this dehydrogenation, cyclohexanone is obtained also.

In the separation, by distillation, of a mixture of cyclohexanone and cyclohexanol obtained from cyclohexane and of a mixture of cyclohexanone and cyclohexanol obtained from phenol, which mixtures may be subjected to this separation jointly if so desired, a distillation residue is obtained in which hardly any cyclohexanone and cyclohexanol are left and which can be converted by combustion, for instance, into a form not harmful to the environment.

It has now been found that from such a distillation residue cyclohexane and methylcyclopentane can be obtained.

The process according to the invention for the preparation of cyclohexane and methylcyclopentane is characterized in that the residue obtained in the separation, by distillation, of a mixture of cyclohexanone and cyclohexanol is subjected to a hydrogenation process in the presence of a hydrocracking catalyst with formation of a reaction mixture containing cyclohexane and methylcyclopentane.

It is noted that the hydrogenation of byproducts obtained in the oxidation of cyclohexane is known from the Japanese patent application 51-118743. This process, however, relates to the hydrogenation of a mixture of high-boiling oils which still contain cyclohexanone and cyclohexanol, while cyclohexene is obtained as product.

The process according to the invention can be performed while using various hydrocracking catalysts known from the petroleum industry, for instance nickle, platinum, palladium, iridium and rhenium on an acid carrier, for instance aluminium silicates, such as zeolites, aluminium oxide and silicon oxide.

Preference is given to performing the process according to the invention with platinum on a zeolite as hydrocracking catalyst.

In the process according to the invention various temperatures can be applied, for instance temperatures ranging from 250 to 550 °C. As a higher temperature is chosen, more methylcyclopentane will be formed. Preferably a temperature of 300-475 °C is applied. If so desired, the process according to the invention can be performed in the presence of a solvant showing an inert behaviour under the reaction conditions.

In the process according to the invention the chosen hydrogen pressure may vary also, for instance a partial hydrogen pressure of 50-300 bar. Preferably a partial hydrogen pressure of 75-150 bar is applied.

After separating off the aqueous phase formed in the hydrogenation process, the reaction mixture obtained according to the invention can be separated by distillation while recovering the cyclohexane and methylcyclopentane formed. If the residu to be converted according to the invention is converted in the presence of a solvent, the solvent used can be recovered in this distillation process and be used again.

Methylcylcopentane can be used, for instance, as solvent in extraction processes. The methylcyclopentane can be converted also into cyclohexane (see, for instance, Houben-Weyl, Methoden der Organischen Chemie 4th edition part 5/1a pages 307-308).

The invention is further elucidated in the following examples.

Example I

Residue obtained in the separation, by distillation, of a mixture of cyclohexanone and cyclohexanol obtained in the oxidation of cyclohexane was pumped from top to bottom through a vertical tube reactor (length 60 cm, diameter 2.3 cm) at a rate of 76 g per hour for 2 hours and 15 minutes. The reactor contained a catalyst bed of platinum on a zeolite as carrier (zeolite PtCaY, containing 0.5 % by weight Pt, commercially available under the name of Union Carbide SK 200) weighing 122 g. The temperature in the reactor was 350 °C, while the partial hydrogen pressure was kept at 100 bar. The residue contained, in addition to high-boiling compounds (boiling point higher than 300 °C), 26 % by weight of a mixture of 2-cyclohexylidene-cyclohexanone and 2-(1-cyclohexenyl)-cyclohexanone, 12 % by weight cyclohexylcyclohexanone, 0.2 % by weight cyclohexanol and less than 0.1 % by weight cyclohexanone.

The reaction mixture obtained in the last 75 minutes was separated into an organic and an aqueous phase. 71 g organic phase and 11 g aqueous phase were obtained. According to a gaschromatographic analysis, the organic phase contained 15.3 % by weight cyclohexane and 5.3 % by weight methylcyclopentane.

The cyclohexane and the methylcyclopentane can be recovered (separately or as a mixture) from this organic phase by distillation.

Example II

Example I was repeated with a throughput of 57 g residue per hour at a temperature of 390 °C under otherwise the same conditions.

In addition to 12 g aqueous phase, 55 g organic phase was obtained containing 15 % by weight cyclohexane and 20 % by weight methylcyclopentane.

Example III

Example I was repeated with a throughput of 99 g per hour at a temperature of 435 °C under otherwise the same conditions.

In addition to 18 g aqueous phase, 103 g organic phase was obtained containing 7 % by weight cyclohexane and 30 % by weight methylcyclopentane.

# C L A I M S

1. Process for the preparation of cyclohexane and methylcyclopentane, characterized in that the residue obtained in the separation, by distillation, of a mixture of cyclohexanone and cyclohexanol is subjected to a hydrogenation process in the presence of a hydrocracking catalyst with formation of a reaction mixture containing cyclohexane and methylcyclopentane.

2. Process according to claim 1, characterized in that as hydrocracking catalyst platinum on a zeolite is used.

3. Process according to claim 1 or 2, characterized in that the hydrogenation is performed at a temperature of 300-475 °C.

4. Process according to any one of claims 1-3, characterized in that the hydrogenation is performed at a partial hydrogen pressure of 75-150 bar.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A | --- <br> CHEMICAL ABSTRACTS, vol. 86, no. 17, 25th April 1977, page 508, no. 120882m, Columbus Ohio (USA); <br> & JP - A - 76 118 743 (UBE INDUS- TRIES LTD.) (18-10-1976) *Abstract* <br><br> ----- | | C 07 C 13/18 <br> C 07 C 13/10 |

TECHNICAL FIELDS
SEARCHED (Int. Cl. ³)

C 07 C 13/00
C 07 C 1/00
C 07 C 4/00

The present search report has been drawn up for all claims

| Place of search <br> THE HAGUE | Date of completion of the search <br> 16-11-1982 | Examiner <br> VAN GEYT J.J.A. |
|---|---|---|